(19) Europäisches Patentamt
European Patent Office
Office européen des brevets

(11) EP 1 801 086 A1

(12) **EUROPEAN PATENT APPLICATION**

(43) Date of publication:
**27.06.2007 Bulletin 2007/26**

(51) Int Cl.:
***C07B 43/06*** *(2006.01)* ***C07C 231/02*** *(2006.01)*

(21) Application number: **05025802.9**

(22) Date of filing: **25.11.2005**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicant: **Synthacon GmbH**
**06237 Leuna (DE)**

(72) Inventors:
- **Kadzimirzs, Daniel**
  **06237 Leuna (DE)**
- **Jas, Gerhard**
  **06237 Leuna (DE)**
- **Autze, Volker**
  **06237 Leuna (DE)**

(74) Representative: **Kador & Partner**
**Corneliusstrasse 15**
**80469 München (DE)**

(54) **Synthesis of carbon acid amides**

(57) The present invention relates to a process for producing carbon acid amides, in particular peptides, whereby the carbon acid amide bond is formed by an activated carboxylic acid and an amine component in a continuous flow. The described process provides an efficient synthesis method for the formation of carbon acid amides, and is applicable even for large scale synthesis ranging from several grams to even more than 100 kg of products.

EP 1 801 086 A1

Printed by Jouve, 75001 PARIS (FR)

## Description

The present invention relates to a process for producing a carbon acid amide.

Formation of carbon acid amides and in particular of peptides is well known in the state of the art and has been further developed over the years.

However, although the synthesis of medium to large peptides for basic research is a well established procedure, the combination of the 20 proteinogenic amino acids and growing number of unnatural amino acids makes each peptide synthesis at the industrial level unique, requiring closer attention to each amino acid coupling.

As regards peptide formation, two different types of peptide synthesis may be distinguished, namely solution phase synthesis and solid phase synthesis. The latter has the advantage that it allows a general synthesis protocol and may easily be automated. However, one of the disadvantages is the fact that a high excess of reactants is necessary in order to allow for complete reactions, making the process as such cost-expensive. Moreover, the scale-up of solid phase synthesis processes is difficult.

On the other hand, solution phase synthesis processes allow for the workup of intermediates. However, the more balanced ratio of starting components and reactants in classical solution phase processes goes at the cost of either reaction time or lower conversion to the desired product. Prolonged reaction time often leads to side reactions, namely epimerisation. To avoid these undesirable effects, higher dilution of the starting components and reactants is necessary, however, this usually results in longer reaction times or lower throughput.

Therefore, recent developments in peptide solution phase synthesis use an excess of one starting material, namely the activated carboxylic component, in order to ensure a rapid and complete amide bond formation, followed by the use of a scavenger in the form of an amine comprising a free anion or a latent anion for quenching the excess reagent. Such processes are described in US 2003/0018163 A1 and US 2003/0018164 A1, respectively.

However, the disadvantage of these processes is the use of an excess of one starting component which makes the process less attractive for large scale synthesis because it requires high amounts of starting materials.

Of course, the above-given advantages and disadvantages of peptide synthesis are not restricted to condensation reactions of α-amino acids, but also apply to the synthesis of carbon acid amides in general, i.e. for reactions of carboxylic acids or derivatives thereof with amines or derivatives thereof.

Accordingly, it is the object of the present invention to provide an efficient synthesis method for the formation of carbon acid amides which overcomes the disadvantages of the state of the art and is applicable for large scale synthesis ranging from grams to even more than 100 kg of product.

It is a further object of the present invention to establish a synthesis method for carbon acid amides and for peptides or isopeptides that avoids epimerisation and/or racemisation.

The present invention is based on the finding that the formation of carbon acid amides may be improved by using a method that provides for an efficient contact of the reaction components during the process.

The key step in carbon acid amide bond formation is the reaction of a carbon acid or derivative thereof, for instance an activated derivative, with an amine component.

Exemplarily, the formation of peptides, which is preferred, will be discussed in the following, as it also shows the principle of protecting group strategies. However, it is known to the skilled person that the starting materials for carbon acid amide formation are not restricted to natural or synthetic α-amino acids, but may further include building blocks with carbon acid and amino functionality that are bound to different carbon atoms of the molecule and even to components bearing either a carbon acid or an amino functionality.

In the following reaction scheme, the procedure of peptide coupling with α-amino acids is demonstrated:

Scheme 1:

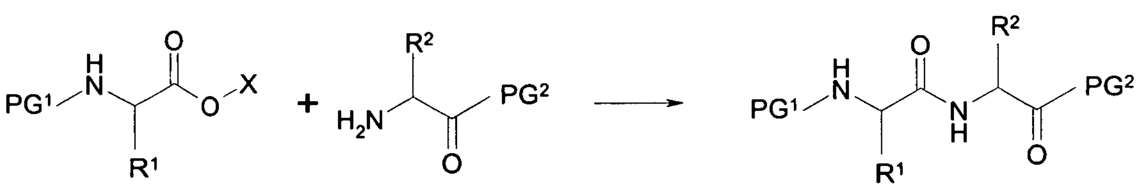

1 2 3

Thereby, as an activated carboxylic acid (1), natural or synthetic amino acid building blocks may be used,

whose further functional groups are either protected or do otherwise not interfere with other components present at the conditions of the coupling reaction.

Protecting groups for amino functionalities, shown in Scheme 1 as $PG^1$, are commonly known in the art. Reference is made to Philip J. Kocienski: "Protecting Groups", 3rd Edition, Georg Thieme, Stuttgart New York, 2004, which gives a good overview of protecting group strategies.

Typical protecting groups for amino functionalities include, but are not restricted to acetyl (Ac), *tert*-butyloxycarbonyl (boc), benzyloxycarbonyl (Z), 9-fluorenylmethoxycarbonyl (fmoc), 2-(methylsulfonyl)-ethoxycarbonyl (Msc), Allyloxycarbonyl (Alloc), 1,1-dioxo-benzo[b]thiophene-2-ylmethoxycarbonyl (Bsmoc) or functions of the arylsulfonyl type, such as *ortho*-nitrobenzenesulfonyl (o-NBS).

$R^1$ and $R^2$ of Scheme 1 designate any side chain of any natural or synthetic α-amino acid. In case these side chains contain functional groups, such groups are protected as known in the art of peptide synthesis, so that they do not interfere with other components present at the conditions of the coupling reaction, and further, that they are stable at the deprotection step of the amino function of the peptide for further chain elongation.

Typical side chain protecting groups include, but are not restricted to *tert*-butyl esters ($^t$Bu), *tert*-butyloxycarbonyl (boc), trityl (Trt), 4-methoxy-2,3,6-phenylsulfonyl (Mtr), 2,2,5,7,8-pentamethylchroman-6-sulfonyl (Pmc), 2,2,4,6,7 - pentamethyldihydrobenzofuran-5-sulfonyl (Pbf), tosyl (Tos), 4-methyltrityl (Mtt), p-methoxytrityl (Mmt), *tert*-butylthio ($^t$Buthio), 4,4'-dimethoxybenzhydrol (Mbh), benzyl ester (OBzl), benzyl (Bzl), acetamidomethyl (Acm), β-1-adamantyl ester (o-1-Ada). Reference is made to Philip J. Kocienski: "Protecting Groups", 3rd Edition, Georg Thieme, Stuttgart New York, 2004, representing a good overview on protecting group strategies for peptide synthesis.

The amine component (2) may be a natural or synthetic amino acid of which the carboxylic acid functionality and functionalities of side chains are protected or do otherwise not interfere with other components at coupling reaction conditions. Moreover, the amine component (2) may be a di- or oligopeptide consisting of natural or synthetic amino acid building blocks which are suitably protected.

$PG^2$ of Scheme 1 may designate a protecting group of the acid functionality. Typical protecting groups include, but are not restricted to methyl ester (OMe), ethyl ester (OEt), tert-butyl ($^t$Bu), 3-(3-methyl-)pentyl (Mpe), 2-(2-phenyl) propyl (Pp), 2-chlortrityl (Clt), diphenyl(4-pyridyl)methyl (PyBzh), Dicyclopropylmethyl (Dcpm), 9-fourenylmethyl (Fm), 2-(trimethylsilyl)ethyl (Tmse), 4-(N-(4,4-dimethyl-2,6-dioxocyclohexylidene)-3-methylbutyl)amino)benzyl (Dmab), benzyl ester (OBzl), benzyl (Bzl) or allyl ester (OAll).

Moreover, $PG^2$ of Scheme 1 may designate at least one further α-amino acid of the peptide chain, whose other functional groups are protected in a manner so that they do not interfere with other components present at the conditions of the coupling reaction.

The activated carboxylic acid may be formed by any activation or coupling agent and coupling additive known in the field. Reference is made to So-Yeop Han, Young-Ah Kim, Tetrahedron 60 (2004) 2447-2467 (and references cited therein) and Peng Li, Peter P.. Roller and Jiecheng Xu: Current Organic Chemistry, 2002, 6, 411-440 where a general overview over modern and current peptide coupling strategies is given.

As an example for an activated carboxylic acid, activated esters such as pentafluorophenyl-, pentachlorophenyl-, 4-nitrophenyl esters may be mentioned, however, any other activated ester suitable and known for peptide synthesis may also be used.

Moreover, activated carboxylic acid species such as halides or azides may be used.

Further, N-carboxyanhydrides such as N-succinimides may be used as activated carboxylic acid.

In principle, the above-mentioned activated esters, halides, azides and N-carboxyanhydrides may also be used as pre-synthesized and isolated preactivated carboxylic acid species.

Moreover, the activated carboxylic acid may be formed *in situ* by the use of carboxylic acid with activating reagents alone or in combination with coupling additives. Such activating reagents and coupling additives are suited to form activated intermediates which subsequently react with the amine component.

Activating reagents include, but are not limited to carbodiimides, 1-hydroxybenzotriazole based or 1-hydroxy-7-azabenzotriazole based phosphonium and uronium salts, halouronium and halophosphonium salts, benzotriazine based uronium salts and phosphates, N-acylimidazoles and N-acyltriazoles.

As representatives for carbodiimides, N,N'-dialkylcarbodiimides such as N,N'-dicyclohexylcarbodiimide (DCC) or diisopropylcarbodiimide (DIPC) may be mentioned. Further, 1-ethyl-3-(3'-dimethylaminopropyl)-carbodiimide (EDC) may be listed.

As representatives for 1-hydroxybenzotriazole based phosphonium salts, benzotriazol-1-yl-*N*-oxy-tris(dimethylamino)phosphonium hexafluorophosphate (BOP), benzotriazol-1-yl-*N*-oxy-tris(pyrrolidino)phosphonium hexafluorophosphate (PyBOP) may be mentioned.

As a representative for 1-hydroxy-7-azabenzotriazole based phosphonium salts, 7-azabenzotriazol-1-yl-*N*-oxy-tris(pyrrolidino)phosphonium hexafluorophosphate (PyAOP) may be mentioned.

As representatives for 1-hydroxybenzotriazole based uronium salts, *N*-[(1*H*-benzotriazol-1-yl)(dimethylamino) methylene]-*N*-methylmethan-aminium hexafluorophosphate *N*-oxide (HBTU), *N*-[(1*H*-benzotriazol-1-yl)(dimethylamino)methylene]-*N*-methylmethanaminium tetrafluoroborate *N*-oxide (TBTU), *N*-[(1*H*-6-chlorobenzotriazol-1-yl)(dimethylamino)-methylene]-*N*-methylmethanaminium hexafluorophosphate *N*-oxide (HCTU), *N*-[(1*H*-6-chlorobenzotriazol-1-yl)(dimethylamino)methylene]-*N*-methylmethanaminium tetrafluoroborate *N*-oxide (TCTU) may be mentioned.

As a representative for 1-hydroxy-7-azabenzotriazole based uronium salts, *N*-[(dimethylamino)-1*H*-1,2,3-triazolo[4,5-*b*]pyridino-aylmethylene]-*N*-methylmethanaminium hexafluorophosphate (HATU) may be mentioned.

As representatives for halouronium salts, bis(tetramethylene)fluoroformamidinium hexafluorophosphate (BTFFH) and 2-chloro-1,3-dimethylimidazolidium hexafluorphosphate may be mentioned.

As representatives for halophosphonium salts, bromotris-(dimethylamino)phosphonium hexafluorophosphate (BroP), bromotripyrrolidino phosphonium hexafluorophosphate (PyBroP) and chlorotripyrrolidino phosphonium hexafluorophosphate (PyCloP) may be mentioned.

As a representative for benzotriazine based uronium salt, *O*-(3,4-dihydro-4-oxo-1,2,3-benzotriazine-3-yl)-*N,N,N',N'*-tetramethyluronium tetrafluoroborate (TDBTU) and 3-(diethyloxyphosphoryloxy)-1,2,3-benzotriazin-4(3*H*)-one (DEPBT) may be mentioned.

As a representative for benzotriazine based phosphonium salt, 3-(diethyloxyphophoryloxy)-1,2,3-benzotriazin-4(3*H*)-one (DEBPT) may be mentioned.

As a representative for an N-acylimidazole, 1,1'-carbonylbis(1*H*-imidazole) (CDI) may be mentioned.

As a representative for an N-acylbenzotriazole, 1,1'-carbonylbis(1*H*-benzotriazole) may be mentioned.

As further activating reagents, 2-bromo-1-ethyl pyridinium tetrafluoroborate (BEP, described in Peng Li, Jie-Cheng Xu: Tetrahedron 56 (2000) 8119-8131) and 2-chloro-4,6-dimethoxy-1,3,5-triazine (CDMT, described in Christine E. Garretts, Xinglong Jiang, Kapa Prasad, Oljan Repic: Tetrahedron Letters 43 (2002) 4161-4165) may be mentioned.

Coupling additives include, but are not limited to 1-hydroxybenzotriazole (HOBt), 1-hydroxy-7-azabenzotriazole (HOAt) and 3,4-dihydro-3-hydroxy-4-oxo-1,2,3-benzotriazine (HO-Dhbt).

The coupling reactions are preferably performed in the presence of a tertiary amine, which includes, but is not limited to triethylamine, ethyldiisopropylamine and N-methylmorpholine.

Again, the principle of reaction scheme 1 may be applied to any carbon acid amide formation and is not restricted to $\alpha$-amino acids.

Thereby, the carbon acid amide formation or the peptide coupling may be performed in inert organic solvents commonly known and used in the art. Preferred solvents are dimethylsulfoxide, N,N-dimethylformamide, N-methylpyrrolidone, dimethylacetamide (DMAC) or mixtures thereof with further organic solvents such as dichloromethane, chloroform, 1,4-dioxane, tetrahydrofuran and ethyl acetate.

The present invention therefore provides a process for producing a carbon acid amide, whereby the carbon acid amide bond is formed by reacting an activated carboxylic acid and an amine component in a continuous flow.

It has been found that when the reaction components are reacted in a continuous flow with each other, they are subjected to an efficient contact to each other during the process, thereby leading to high conversion rates.

The efficient contact results from the fact, that in continuous flow systems the reaction volume can be small, compared with the total produced output volume.

In the conventional synthesis with increasing the output volume, the vessel size has to be increased and even with constant reaction times, normally at least the adding time of reagents has to be prolonged. Due to less efficient contact of the reaction components in the case of increasing the vessel size, very often also the pure reaction time has to be prolonged.

Using continuous flow enables the control of the reaction time independently from the output volume simply by feeding raw materials longer into the reaction system. The reaction time is always determined by the ratio of reaction volume and flow rate. Increasing the concentration leads to further acceleration of the reaction, which enables to choose further shortened reaction times. By this, continuous flow yields a greatly enhanced throughput in general and when $\alpha$-amino-acids are reacted, it prevents further the occurrence of epimerisation.

The continuous flow is achieved by feeding the starting materials or starting compounds simultaneously into the reaction system and by withdrawing the chemical product out of the reaction system.

Preferably, the starting materials or starting compounds and the effluent materials are fed simultaneously into and respectively out of the reaction system.

More preferred, the reaction system is completely filled with a stream of starting materials or starting compounds and a stream of effluent materials.

Preferably, the activated carboxylic acid and the amine component are fed, each in a continuous flow, simultaneously into the reaction system.

Thereby, the flow rates for each component may be the same or different from each other, depending on the concentration of the components or on their balance in the reaction.

Preferably, the activated carboxylic acid and the amine component are present in an inert liquid.

Thereby, the activated carboxylic acid and the amine component and/or the respective reaction product may form a solution, a suspension or a dispersion with the inert liquid.

As liquid, dimethylsulfoxide, N,N-dimethylformamide, N-methylpyrrolidone, dimethyl-acetamide (DMAC) or mixtures thereof with further organic solvents such as dichloromethane, chloroform, 2,2,2-trifluoroethanol, 1,4-dioxane, tetrahydrofuran and ethyl acetate are preferably used.

The efficient contact of the reaction components found for the continuous flow processes can be further improved by especially focussing on mixing.

Preferably, the activated carboxylic acid and the amine component are fed into a system bearing at least one tube or channel, wherein the components are mixed and pass through the channel, thereby forming the respective amide bond.

Preferably, the channels have a hydraulic diameter of 50 μm to 2 mm, more preferably from 100 μm to 1,5 mm, and most preferably the channels have a hydraulic diameter of 150 μm to 1 mm.

The term hydraulic diameter indicates that various geometries of the cross-sectional area of the channel or tube may be used, i.e. the method according to the invention is independent from a specific geometry of the cross-sectional area of the channel or tube.

The hydraulic diameter $D_h$ is defined as follows

$$D_h = \frac{4A}{U}$$

whereby A is the cross-sectional area and U is the wetted perimeter of the cross-section.

Preferably, the reaction system is capable of performing mixing and heat exchanging in the same device.

Preferably, mixing is achieved by diffusion. Most preferably, mixing is achieved via multilamination of the starting materials or starting compounds by creating alternating layers of starting materials, with a layer thickness less than the height of the channel.

It has been found that the spatial constitution or geometry of a synthesis reactor contributes to an improved reaction kinetic, i.e. due to perfect contact of each reaction component to each other, very high conversion rates are achieved in carbon acid amide or peptide coupling reactions. Thereby, the starting materials or starting compositions, namely the activated carboxylic acid and the amine component, are fed in a continuous flow into a channel preferably having a hydraulic diameter of 50 μm to 2 mm, which allows for a rapid reaction between the components with high conversion rate and essentially pure products.

The hydraulic diameter of the channel may thereby even be smaller, i.e. from 100 μm to 1,5 mm or from 150 μm to 1 mm.

The continuous flow of the starting materials into the reaction system or channel causes a continuous output of the product. Thereby, the length of the reaction channel may be varied, depending on the specific components to be reacted, in order to allow for a complete conversion to the desired product. At a given volume of the channel, the flow rate determines the residence time of the reaction mixture in the channel system.

In a further embodiment of the invention, the length of the reaction channel may be extended by a further module containing an extension channel in order to define a specific residence time of the reaction mixture in the channel system. The extension channel may have the same hydraulic diameter as given above and as used for the reaction channel.

Preferably, the process is conducted in a way that the residence time of the components or the mixture of starting material and products in the reaction channel or the reaction channel and the extension channel is less than 90 min, preferably less than 45 min, preferably less than 30 min and preferably less than 5 min, however, at least 1 sec.

Preferably, the activated carboxylic acid and the amine component are reacted in a ratio of 1.2:1 to 1:1.2, preferably in a ratio of 1.1:1 to 1:1.1, preferably from 1.05:1 to 1:1.05, and more preferably in a ratio of 1.01:1 to 1:1.01. Also, a ratio of 1:1 is possible with the effective reaction system according to the invention. Thereby, the intensive contact of the reaction partners allows for high conversion rates within short time frames.

The starting concentration of the reaction components, i.e. the carboxylic acid derivative and the amine component may be, from saturation of the reaction component in the given solvent as a highest concentration, down to 0.01 mol/1 as a lowest concentration, preferably from 1.0 mol/l to 0.05 mol/l, more preferably from 0.5 mol/l to 0.1 mol/l, and most preferably from 0.3 to 0.15 mol/l.

The preferred or most preferred concentrations in combination with short reaction times allow for a high throughput and high conversion rates from starting material to product, and consequently for large scale synthesis of carbon acid amides, peptides or isopeptides.

The process according to the invention may be performed at any temperature known and commonly used in the field. However, temperatures from -20°C to 60°C are preferred. Advantageously, the temperature is kept at 20 to 60°C, and most preferably, it is kept at 30 to 50°C. Such relatively high temperature allow for a fast reaction and hence

for low residence time, which is preferred for large scale synthesis. However, the process of the present invention thereby suppresses or even avoids epimerisation of the components, which would be expected to be cause relevant amounts of side products at such temperatures.

In traditional peptide synthesis in a round bottom flask 3-(diethoxyphosphoryloxy)-1,2,3-benzotriazin-4(3*H*)-one (DEPBT) is an attractive alternate activating agent. Unfortunately, the reaction is slow and attempts to accelerate the reaction by rising the reaction temperature lead to significant epimerisation, e.g. in Pro-His-coupling reactions at -15°C no epimerisation occurs, at 20°C about 1-5% epimerisation is obtained and at 60°C, complete epimerisation is observed (M.Mergler, et al. (2001) J.Pept. Sci.,7, 502.

The continuous flow of the reaction partners is typically achieved via mechanical pumping or pressure. Therefore, mechanical pumps are used to build up and maintain the flow in the reaction system. As an example for mechanical pumps, piston pumps, peristaltic pumps, syringe pumps or diaphragm pumps may be mentioned. Hydrostatic and pneumatic pumps may be mentioned as an example for pressure. Thereby, hydrostatic systems may involve reservoirs with liquids that are passed through the system by their own pressure.

In principle, any transfer rate through the reaction system, and in particular through the reaction channel itself may be kept at 0.005 ml/min to 40 ml/min when concentrations as given above are used, namely from saturation of the reaction component in the given solvent as a highest concentration down to 0.01 mol/l as a lowest concentration or from 1.0 mol/l to 0.05 mol/l.

Preferably, the transfer rate through one reaction system, and in particular through the reaction channel itself may be 0.005 ml/min to 40 ml/min when the concentration of the solutions is kept at 0.1 to 0.5 mol/l.

With the assumption of a molecular weight of 300 an example for the production capacity of one reaction system is given in the following table:

Table I:

| Production period | Transfer rate with 0,5 Mol/l | Product quantity Mol | Product quantity kg |
|---|---|---|---|
| Hour | 10 ml/min | 0,3 Mol/h | 0,09 kg/h |
| Day | 10 ml/min | 7,2 Mol/d | 2,16 kg/d |
| Year | 10 ml/min | 2400 Mol/y | 720 kg/y |
| | | | |
| Hour | 20 ml/min | 0,6 Mol/h | 0,18 kg/h |
| Day | 20 ml/min | 14,4 Mol/d | 4,32 kg/d |
| Year | 20 ml/min | 4800 Mol/y | 1440 kg/y |
| | | | |
| Hour | 40 ml/min | 1,2 Mol/h | 0,36 kg/h |
| Day | 40 ml/min | 28,8 Mol/d | 8,64 kg/d |
| Year | 40 ml/min | 9600 Mol/y | 2880 kg/y |

Preferably, the reaction channel as mentioned above forms a part of a microreactor. Further, the microreactor may be supplemented by a module for extending the residence time. Moreover, the microreactor may be supplemented with a first and/or second premixer.

Moreover, more than one microreactor may be operated simultaneously, i.e. in parallel in order to achieve large scale production of carbon acid amides or peptides, in particular industrial scale production. The advantage of using an assembly of parallel operated microreactors is to avoid time consuming scale-up of reactions, but rather use a numbering up of already optimized reaction processes. Another advantage is the easier control of reaction temperature and reaction time of the process.

Preferably, the activated carboxylic acid according to the process of the invention is formed by reaction of a carboxylic acid and an activating agent. Thereby, the activating agent may contain or comprise a coupling agent.

Preferably, the process according to the present invention comprises a premixing step prior to the formation of the amide bond.

Thereby, a carboxylic acid and an activating agent are mixed in a first premixer. It has been surprisingly found that when a premixing step is involved, the racemization or epimerisation in amide or peptide bond formation may further be effectively suppressed or avoided.

It is preferred that the stagnant volume in the premixer is kept as small as possible. Thereby the premixer may

consist of any equipment capable to mix two or more liquids. For instance, the premixer may consist of a T-piece. T-pieces are commonly used in chemistry, for instance in HPLC techniques. For the present invention, 1/16" T-pieces may preferably be used. On the other hand, the premixer may also consist of a microreactor.

Preferably, the carboxylic acid and the activating agent are fed in a continuous flow and separately from each other via inlet channels into a first premixer equipped with a mixing channel where the mixing essentially takes place.

If desired, a coupling additive may be added to the activating agent.

Thereby, the activated carboxylic acid may be formed in the mixing channel of the first premixer. Alternatively, it may be *in situ* formed in the reaction channel subsequent to the mixing channel of the first premixer.

Alternatively, an activated carboxylic acid and a coupling additive may be mixed in a first premixer. Again, the premixer comprises inlet channels and a mixing channel.

Preferably, the first premixer is located directly before the reaction channel so that the mixing of carboxylic acid with activating agent and activating agent together with coupling additive, respectively, is achieved instantaneously before the carbon acid amide or peptide coupling itself. Thereby, instantaneously is meant in a timely and spatial manner.

Moreover, prior to the formation of the carbon acid amide bond, the amine component and a base may be mixed in a second premixer. Preferably, the amine component and the base are fed in a continuous flow and separately from each other via inlet channels into a second premixer equipped with a mixing channel where the mixing essentially takes place.

Again, the second premixer is preferably located directly before the reaction system where carbon acid amide or peptide coupling takes place.

For the premixing, the carboxylic acid, the activating agent, the coupling additive, the amine component and the case may be fed into the respective premixers in form of a solution, a suspension or a dispersion.

The work up of the products, i.e. carbon acid amides, peptides or isopeptides is effected by leading the continuous flow of product into an aqueous solution of NaCl, for instance at a concentration of 5wt%. Alternatively, water or any other common buffer may be used.

Subsequently, the collected product suspension is extracted with organic solvent which is immiscible with water. Preferably, organic solvents such as ethyl acetate, dichloromethane or methyl-tert-butyl ether are used. The organic phases may then be re-extracted with aqueous solution of salt, preferably NaCl, $NaHCO_3$, $Na_2CO_3$ or citric acid. After drying the organic phase with commonly known drying agents, preferably with $MgSO_4$ or $Na_2SO_4$, the resulting product is separated from the solvent, preferably by evaporation of the solvent under reduced pressure.

Usually, no further purification step such as chromatographic purification is necessary as the process according to the invention allows for the production of essentially pure products.

In case of peptide or isopeptide synthesis, the bond formation step is then followed by a subsequent deprotection step of either the protected amine or the acid functionality. Respective deprotection methods are known in the field. Reference is made to Philip J. Kocienski: "Protecting Groups", 3rd Edition, Georg Thieme, Stuttgart New York, 2004 and references cited therein. Deprotection may either be performed by reacting the protected peptide or isopeptide with deprotecting reagent in a continuous flow. Preferably, the protected peptide or isopeptide and the deprotecting reagent are fed, each in a continuous flow, simultaneously into the reaction system. Preferably, both, the protected peptide or isopeptide and the deprotecting reagent, respectively, are present in an inert liquid, thereby forming a solution, a suspension or a dispersion. The reaction system may be the same as described above.

Alternatively, the deprotection step may be performed in a batch reaction, i.e. in a commonly used reaction vessel such as glass equipment at a size ranging from 250 ml to 100 1.

For deprotection of the *tert*-butyloxycarbonyl (boc) protecting group, formic acid is preferably used.

Final deprotection may be achieved by deprotecting steps commonly known in the field. Reference is made to Peng Li, Peter P. Roller and Jiecheng Xu: Current Organic Chemistry, 2002, 6, 411-440. Preferably, a mixture of trifluoro acetic acid (in most cases diluted with dichloromethane) in the presence of scavenging reagents like thioanisol, DTT (dithiothreitol) is used.

In the following examples, the principle of the inventive will be shown by a synthesis of a nonapeptide.

## Equipment

For all reactions a CYTOS® Lab System (CPC-Systems GmbH, Mainz, Germany) equipped with a CYTOS® microreactor, additional residence time units in order to realize suitable residence times (see experimental details) and a thermostating unit (Huber) was used. Two syringe pumps devices equipped with two 10 ml syringes each (Kloehn) were used for feeding the raw materials in the reactor. Conventional 1/16" T-pieces (junction) as used for HPLC systems and an additional pump were chosen for achieving the premixing, if necessary. The syringe pumps maybe replaced by rotary piston pumps (Ismatec).

## Analytics

All reactions were monitored by HPLC using a HP 1100 (Hewlett-Packard) system, equipped with a UV detector capable of monitoring at 205 nm, an injector capable to inject 20 μL and suitable integrating device. For the whole synthesis sequence a linear gradient was run according the following scheme:

| | | |
|---|---|---|
| Column: | Xterra RP18 5μm, 4.6 x 250mm | |
| Eluent: | A = 0.1 % TFA in water - B = 0.1 % in acetonitrile | |
| Flow: | 1.5 ml/min | |
| Gradient: | 0 min | 8% B |
| | 10 min | 65% B |
| | 16 min | 100% B |
| | 18 min | 100% B |
| | 18.01 min | 8% B |
| | 23 min | 8% B |

## General procedure for HBTU promoted coupling

All materials required for the reaction are dissolved in anhydrous DMF or DMAC. A total of four storage containers which are kept under argon, are individually filled with the acid component used, HBTU as coupling reagent, the amino compound and triethylamine (DIPEA in case of Fmoc protecting groups present in the coupling compounds). Concentrations are preferably in the range of about 0.2 mol/l and the CYTOS® Lab System is set to the suitable reaction temperature by the external thermostating unit. The pumps are initialised and the system is filled with the appropriate solvent (DMAC or DMF) from a separate vessel. After the system is filled with DMF (peptide grade), the containers with the starting material solutions are fitted with the system and the premixed solutions (A+B) and (C+D) fed into the reactor by separate inlet pipes using flow rates required to achieve the appropriate residence times. A well stirred vessel with 5% sodium chloride solution cooled with an ice bath is used for product collection. At 1.5 τ a sample for IPC by HPLC is taken in order to determine a) if the steady state of the system has been reached and b) if the reaction shows the appropriate turnover. If needed, the pumps are calibrated again. After having consumed all starting materials the inlet feed is switched to solvent (dry DMAC or DMF) and product collection is continued for further 1.5 inner system volumes of reaction mixture. Then the product valve is switched to waste and the system is cleaned with solvent followed by water and ethanol.

The collected product suspension is extracted with a mixture of ethylacetate and MTBE for two or three times. The combined organic layers are washed with 0.5 M citric acid (pH control), then with 5% sodium chloride solution, 10% $NaHCO_3$ solution, again with 5% sodium chloride solution and finally with concentrated brine. After drying the combined organic phases over $MgSO_4$ the solvent is removed under reduced pressure (bath temperature max. 30 °C) yielding the crude product. The content (yield) of the product is determined by HPLC analysis.

## General procedure for Boc-cleavage

The crude material of the coupling step is dissolved in cold formic acid and stirred under argon-atmosphere at room temperature unto completion (HPLC control). Subsequently, the formic acid is removed at 35 °C under reduced pressure. To ensure the complete removal of the acid the residue is co-evaporated several times with toluene and evaporated to dryness. Finally desalting is achieved using a DOWEX 1-X8 ion exchange resin.

## General procedure for Fmoc-cleavage

In an appropriate round bottomed flask equipped with a dropping funnel the peptide is dissolved in ethyl acetate and cooled to 0°C. A solution of 1.5 equivalents of TAEA (tris aminoethyl amine) in ethyl acetate is added dropwise and the mixture stirred for additional 30 min unto completion of the reaction. The tenfold amount of ethyl acetate is added and the slurry treated in an ultrasonic bath for 5 min. The precipitate is filtered off and the filtrate extracted four times with 5% sodium chloride solution and additionally with concentrated brine. After drying over 30 g of magnesium sulfate the solvent is evaporated (at 20 °C) to a small volume of - 30 ml. The product content is determined by HPLC. The solution is directly used in the next coupling step.

**General procedure for saponification**

A 0.6 M solution of crude ester in ethanol is cooled down to 0 °C and a solution of 10 equivalents of 10N NaOH in water is added drop wise and the mixture is stirred for 2 h or unto completion of the reaction. 2/3 parts of the ethanol are removed under reduced pressure at 30 °C and dichloro methane is added followed by 1 M citric acid. The phases are separated and the aqueous layer is extracted twice with dichloro methane. The combined organic layers are washed with 5 % sodium chloride solution and with 100 ml of conc. brine. After drying with $MgSO_4$ the solvent is removed under reduced pressure and the residue is dried in vacuum.

**Boc-Arg(Mtr)-Pro-NHEt**

According to the general procedure for HBTU promoted coupling solutions of 31.5 g Boc-Arg(Mtr)-OH in DMAC, 24.79 g HBTU in DMAC, 11.56 g H-Pro-NHEt•HCl in DMAC and 19.60 g triethylamine in DMAC were premixed and fed into the reactor at 50°C using a residence time of 1.1 min to yield 52 g crude material which was used in the next step without purification. The calculated yield from HPLC analysis was 99%.

**Arg(Mtr)-Pro-NHEt•HCOOH**

According to the general procedure for Boc-cleavage 52 g Boc-Arg(Mtr)-Pro-NHEt (0.38 M in DMAC) were treated with 250 ml formic acid to yield 39 g product (90% yield based on HPLC analysis) as formiate. Finally the material was desalted using a Dowex 1-X8 ion exchange resin.Boc-D-Leu-**Leu-OAllyl:**

According to the general setup of coupling reactions a solution of 21.03 g of Boc-D-Leu-OSu in 126 ml of dry DMF and a solution of 22.0 g H-Leu-OAll•HOTos and 19.4 g triethylamine in 128 ml dry DMF was fed into the microreactor at 50°C using a CYTOS® Lab System equipped with two additional residence time units (15 ml each) at 60°C using a total flow of 3.2 ml/min resulting in a residence time of 10 min. The product was collected and isolated as usual to yield 22.9 g of crude product which was used in the next reaction without purification.

**Boc-D-Leu-Leu-OH**

According to the general saponification procedure, 6.11 g crude Boc-D-Leu-Leu-OAllyl were saponified with 3 g sodium hydroxide in ethanol/water to yield 5.0 g crude Boc-D-Leu-Leu-OH which was used in the next step without purification

**Boc-D-Leu-Leu-Arg(Mtr)-Pro-NHEt**

According to the general procedure for HBTU promoted coupling, 14.0 g Boc-D-Leu-Leu-OH, 20.7 g Arg (Mtr)-Pro-NHEt, 16.6 g HBTU, 9.3 ml triethylamine were premixed and reacted in a CYTOS® Lab System in anhydrous DMF at 40°C and a residence time of 2 min at a total flow rate of 23,5 ml/min to yield 18.2 g crude material (yield based on HPLC analysis: 60%) which was used in the next step without purification.

**D-Leu-Leu-Arg(Mtr)-Pro-NHEt**

According to the general procedure for BOC-cleavage, 500 mg crude Boc-D-Leu-Leu-Arg(Mtr)-Pro-NHEt was treated with 2 ml formic acid yielding 350mg crude D-Leu-Leu-Arg(Mtr)-Pro-NHEt (yield based on HPLC: 80%).

**Pyr-His(Trt)-OMe**

According to the general procedure for HBTU promoted coupling, 4.7 g (10.5 mmol) H-His(Trt)-OMe • HCl, 4.54 g (10.8 mmol) HBTU, 2.69 g (10.5 mmol) Pyr-OH and 8.3 ml (26.3 mmol) DIPEA were reacted in a CYTOS® Lab System in anhydrous DMF at total flow of 26.7 ml/min and a residence time of 1.2 min to yield 5,3 g of crude Pyr-His (Trt)-OH which was used in the next step without purification. Yield based on HPLC: 81%.

**Pyr-His(Trt)-OH**

According to the general procedure for saponification, 5.6 g crude Pyr-His(Trt)-OMe were treated with 3.6 g sodium hydroxide in ethanol/water yielding 5.6 g of crude material (yield based on HPLC:99 %)

**Fmoc-Ser($^t$Bu)-Tyr($^t$Bu)-OAllyl**

According to the general procedure for HBTU promoted coupling, 18.26 g Fmoc-Ser(tBu), 16.95 g HBTU, 11.95 g H-Tyr(tBu)-OAllyl and 26.10 ml DIPEA were reacted in a CYTOS® Lab System in anhydrous DMF at 45°C and at a total flow rate of 32 ml/min at residence time of 1 min to yield 26.09 g crude material (yield based on HPLC: 96%) which was used in the next step without purification.

**Ser($^t$Bu)-Tyr($^t$Bu)-OAllyl**

According to the general procedure for Fmoc-cleavage, 9 g Fmoc-Ser($^t$Bu)-Tyr($^t$Bu)-OMe, 22 ml TAEA in ethyl acetate yielded 4.7 g of crude product (yield based on HPLC: 88%).

**Fmoc-Trp(boc)-Ser($^t$Bu)-Tyr($^t$Bu)-Oallyl**

According to the general procedure for HBTU promoted coupling, crude H-Ser($^t$Bu)-Tyr($^t$Bu)-OAllyl, freshly Fmoc-cleavaged from 33.3 g of Fmoc-Ser($^t$Bu)-Tyr($^t$Bu)-OAllyl were reacted in a CYTOS® Lab System with 27.3 g of Fmoc-Trp(boc)-OH, 20.15g HBTU and 17.7 ml of DIPEA in anhydrous DMF yielding 49.2 g crude product (yield based on HPLC: 91%).

**Trp(boc)-Ser($^t$Bu)-Tyr($^t$Bu)-Oallyl**

49.2 g of crude Fmoc-Trp(boc)-Ser($^t$Bu)-Tyr($^t$Bu)-OAllyl were treated with 9.2 ml of TAEA as described in the general procedure for Fmoc-cleavage yielding 45.3 g of crude product (yield based on HPLC: 98%), which is subsequently used for the next coupling.

**Pyr-His(Trt)-Trp(boc)-Ser($^t$Bu)-Tyr($^t$Bu)-Oallyl**

Solutions of 45.3 g of crude Trp(boc)-Ser($^t$Bu)-Tyr($^t$Bu)-OAllyl in 80 ml of anhydrous DMF, and 22.0 g of Pyr-His(Trt)-OH in 120 ml of anhydrous DMF are prepared and combined in a vessel. After cooling to -15°C a solution of 14.86 g of DEPBT and 9.34 g of HOBT•$H_2O$ in 50 ml of anhydrous DMF is added and stirred for additional 15 min. Finally, a solution of 10.5 ml DIPEA in 10 ml of anhydrous DMF is added and the mixture stirred for 12 hrs at -15°C. After workup similar to the description in the general coupling procedure, 49.16 g crude material are obtained. The calculated yield from HPLC analysis was 78 %.

**Pyr-His(Trt)-Trp(boc)-Ser($^t$Bu)-Tyr($^t$Bu)-OH**

According to the general saponification procedure, 6.34 g crude Pyr-His(Trt)-Trp(boc)-Ser($^t$Bu)-Tyr($^t$Bu)-OAllyl were saponified with 10.8 ml of 1 M sodium hydroxide in ethanol/water. After aqueous work up, the crude product is redissolved in dichloro methan and MTBE is added. The precipitate is filtered off and dried in vacuum, yielding 5.95 g of Pyr-His(Trt)-Trp(boc)-Ser($^t$Bu)-Tyr($^t$Bu)-OH (yield based on HPLC: 94%)

**Pyr-His(Trt)-Trp(boc)-Ser($^t$Bu)-Tyr($^t$Bu)-D-Leu-Leu-Arg(Mtr)-Pro-NHEt**

According to the general procedure for HBTU promoted coupling, 7.43 g of crude D-Leu-Leu-Arg(Mtr)-Pro-NHEt were reacted in a CYTOS® Lab System with 11.64 g of Pyr-His(Trt)-Trp(boc)-Ser($^t$Bu)-Tyr($^t$Bu) and 3.25 ml of DIPEA. Differing from the general procedure, 7.41 g of PyBOP and 1.92 g of HOBT are used for the reaction instead of HBTU. For the reaction a temperature of 35°C and a residence time of 40 min at a total flow rate of 3 ml/min is adjusted. After aqueous work up, 19.7 g of crude Pyr-His(Trt)-Trp(boc)-Ser($^t$Bu)-Tyr($^t$Bu)-D-Leu-Leu-Arg(Mtr)-Pro-NHEt are isolated. The calculated yield from HPLC analysis was 68 %.

**Pyr-His-Trp-Ser-Tyr-D-Leu-Leu-Arg-Pro-NHEt**

14 g of crude Pyr-His(Trt)-Trp(boc)-Ser(tBu)-Tyr(tBu)-D-Leu-Leu-Arg(Mtr)-Pro-NHEt are dissolved in 60 ml of dichloro methan and 17 g of DTT are added, followed by 150 ml of TFA. The mixture is stirred under reflux for 6 h and then the solution is poured into 450 ml of MTBE. The precipitate is filtered, redissolved in water and purified by chromatography using carboxymethyl cellulose and a gradient of water and 0.2 M aqueous ammonium acetate solution as eluent. 7.1 g of product is isolated with a calculated yield from HPLC analysis of 59%.

## Claims

1. A process for producing a carbon acid amide, **characterized in that** the carbon acid amide bond is formed by reacting an activated carboxylic acid and an amine component in a continuous flow.

2. A process according to claim 1, **characterized in that** the activated carboxylic acid and the amine component are fed, each in a continuous flow, simultaneously into a reaction system

3. A process according to claim 1 or 2, **characterized in that** the activated carboxylic acid and the amine component are present in an inert liquid.

4. A process according to any of claims 1 to 3, **characterized in that** the activated carboxylic acid and the amine component and/or the reaction product form a solution, a suspension or a dispersion with the inert liquid.

5. A process according to any of claims 1 to 4, **characterized in that** the activated carboxylic acid and the amine component are fed into a system bearing at least one channel, wherein the components are mixed and passed through the channel, thereby forming the respective amide bond.

6. A process according to claim 5, **characterized in that** the channel has a hydraulic diameter of 50 μm to 2 mm, preferably of 100 μm to 1.5 mm, and most preferably from 150 μm to 1 mm.

7. A process according to claim 5 or 6, **characterized in that** the reaction channel is extended by a further module containing an extension channel.

8. A process according to claim 1 to 7, **characterized in that** the activated carboxylic acid and the amine component are reacted in a ratio of 1.2:1 to 1:1.2, preferably in a ratio of 1.1:1 to 1:1.1, still more preferably in a ratio of 1.01:1 to 1:1.01.

9. A process according to any of claims 5 to 8, **characterized in that** the residence time of the components in the channel according to any of claims 5 to 7 is less than 90 min, preferably less than 30 min, more preferably less than 10 min and most preferably less than 5 min.

10. A process according to any of claims 1 to 9, **characterized in that** the starting concentration of the carboxylic component and the amine component is from saturation of the carboxylic component and/or the amine component in a given solvent to 0.01 mol/l, preferably from 1.0 mol/l to 0.05 mol/l, more preferably from 0.5 mol/l to 0.1 mol/l, most preferably from 0.3 mol/l to 0.15 mol/l.

11. A process according to any of claims 1 to 10, **characterized in that** the temperature in the channel system is kept in a range from -20 to 60°C, preferably from 20 to 60°C, and most preferably from 30 to 50°C.

12. A process according to any of claims 1 to 11, **characterized in that** the channel of claim 1 is part of a microreactor.

13. A process according to claim 12, **characterized in that** more than one microreactors are operated in parallel.

14. A process according to any of claims 1 to 13, **characterized in that** mechanical pumping or pressure is used for the generation of a continuous flow.

15. A process according to any of claims 1 to 14, **characterized in that** the activated carboxylic acid is formed by reaction of a carboxylic acid and an activating agent.

16. A process according to claim 15, **characterized in that** the activating agent contains a coupling agent.

17. Process according to any of claims 1 to 16, **characterized in that** prior to the formation of the carbon acid amide bond, a carboxylic acid and an activating agent are fed in a continuous flow and separately from each other via inlet channels into a first premixer equipped with a mixing channel where the mixing essentially takes place.

18. Process according to claim 17, **characterized in that** the activating agent additionally contains a coupling agent.

**19.** Process according to claims 17 or 18, **characterized in that** the activated carboxylic acid is formed within the mixing channel of claim 17.

**20.** Process according to any of claims 1 to 16, **characterized in that** prior to the formation of the carbon acid amide bond, an activated carboxylic acid and a coupling agent are fed in a continuous flow and separately from each other via inlet channels into a premixer equipped with a mixing channel where the mixing essentially takes place.

**21.** Process according to any of claims 1 to 20, **characterized in that** prior to the formation of the amide bond, the amine component and a base are fed in a continuous flow and separately from each other via inlet channels into a second premixer equipped with a mixing channel where the mixing essentially takes place.

European Patent Office

# EUROPEAN SEARCH REPORT

Application Number
EP 05 02 5802

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | US 5 905 167 A (DITRICH ET AL)<br>18 May 1999 (1999-05-18)<br>* column 1, lines 44-48 *<br><br>* column 4, lines 58-67 *<br>* column 5, lines 1-11; example 1 *<br>----- | 1-5,8,9,<br>11,15,16<br>6,7,10,<br>12-14,<br>17-21 | INV.<br>C07B43/06<br>C07C231/02 |
| Y | DE 103 03 581 A1 (CLARIANT GMBH)<br>12 August 2004 (2004-08-12)<br>* paragraphs [0006] - [0008], [0021], [0022], [0028], [0031], [0034], [0035], [0037] - [0040]; examples 3,4 *<br>----- | 1-21 | |
| A,D | US 2003/018164 A1 (EGGEN IVO FRANCI ET AL)<br>23 January 2003 (2003-01-23)<br>* the whole document *<br>----- | 1-21 | |
| Y | DEWITT S H: "Microreactors for chemical synthesis"<br>CURRENT OPINION IN CHEMICAL BIOLOGY, CURRENT BIOLOGY LTD, LONDON, GB,<br>vol. 3, 1999, pages 350-356, XP002257813<br>ISSN: 1367-5931<br>"Introduction" on page 350; Table 1 and "Critical analysis", 1st paragraph on pag 354<br>----- | 1-21 | TECHNICAL FIELDS SEARCHED (IPC)<br>C07B<br>C07C<br>C07K |

The present search report has been drawn up for all claims

| Place of search | Date of completion of the search | Examiner |
|---|---|---|
| Munich | 17 May 2006 | Seelmann, M |

1

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO.

EP 05 02 5802

This annex lists the patent family members relating to the patent documents cited in the above-mentioned European search report.
The members are as contained in the European Patent Office EDP file on
The European Patent Office is in no way liable for these particulars which are merely given for the purpose of information.

17-05-2006

| Patent document cited in search report | | Publication date | Patent family member(s) | | | Publication date |
|---|---|---|---|---|---|---|
| US 5905167 | A | 18-05-1999 | AT | 191451 | T | 15-04-2000 |
| | | | CA | 2229312 | A1 | 20-03-1997 |
| | | | CN | 1196046 | A | 14-10-1998 |
| | | | DE | 19534208 | A1 | 20-03-1997 |
| | | | WO | 9710201 | A1 | 20-03-1997 |
| | | | EP | 0850214 | A1 | 01-07-1998 |
| | | | ES | 2145485 | T3 | 01-07-2000 |
| | | | JP | 11512411 | T | 26-10-1999 |
| | | | PT | 850214 | T | 29-09-2000 |
| | | | TW | 408097 | B | 11-10-2000 |
| DE 10303581 | A1 | 12-08-2004 | CN | 1745056 | A | 08-03-2006 |
| | | | WO | 2004067492 | A1 | 12-08-2004 |
| | | | EP | 1590315 | A1 | 02-11-2005 |
| US 2003018164 | A1 | 23-01-2003 | AT | 302792 | T | 15-09-2005 |
| | | | BR | 0202783 | A | 10-06-2003 |
| | | | CA | 2390358 | A1 | 19-01-2003 |
| | | | CN | 1398876 | A | 26-02-2003 |
| | | | DE | 60205693 | D1 | 29-09-2005 |
| | | | DK | 1291356 | T3 | 19-12-2005 |
| | | | ES | 2248485 | T3 | 16-03-2006 |
| | | | HK | 1050904 | A1 | 21-10-2005 |
| | | | HR | 20020609 | A1 | 28-02-2003 |
| | | | JP | 2003055396 | A | 26-02-2003 |
| | | | MX | PA02006998 | A | 17-02-2005 |
| | | | NO | 20023446 | A | 20-01-2003 |
| | | | PL | 355125 | A1 | 27-01-2003 |
| | | | PT | 1291356 | T | 30-11-2005 |
| | | | RU | 2237673 | C2 | 10-10-2004 |
| | | | SG | 119160 | A1 | 28-02-2006 |
| | | | ZA | 200205409 | A | 05-09-2002 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20030018163 A1
- US 20030018164 A1


### Non-patent literature cited in the description


- **PHILIP J. KOCIENSKI.** Protecting Groups. Georg Thieme, 2004
- **SO-YEOP HAN ; YOUNG-AH KIM.** *Tetrahedron,* 2004, vol. 60, 2447-2467
- **PENG LI ; PETER P.. ROLLER ; JIECHENG XU.** *Current Organic Chemistry,* 2002, vol. 6, 411-440
- **PENG LI ; JIE-CHENG XU.** *Tetrahedron,* 2000, vol. 56, 8119-8131
- **CHRISTINE E. GARRETTS ; XINGLONG JIANG ; KAPA PRASAD ; OLJAN REPIC.** *Tetrahedron Letters,* 2002, vol. 43, 4161-4165
- **M.MERGLER et al.** *J.Pept. Sci.,* 2001, vol. 7, 502
- **PENG LI ; PETER P. ROLLER ; JIECHENG XU.** *Current Organic Chemistry,* 2002, vol. 6, 411-440